# EUROPEAN PATENT APPLICATION

(11) **EP 4 649 931 A1**
(43) Date of publication of application: **19.11.2025**
(21) Application number: 25175125.1
(22) Date of filing: 08.05.2025
(51) Int. Cl.: A61G 7/057, F16K 15/20, F16K 17/04, F16K 37/00, F04B 33/00, F04B 39/12

(54) **A PRESSURE RELIEVING VALVE ASSEMBLY, INFLATABLE SUPPORT ASSEMBLY AND A METHOD OF INFLATING AN INFLATABLE SUPPORT MEMBER**

(30) Priority: 13.05.2024 GB 202406706
(71) Applicant: Frontier Therapeutics Limited, Blaenau Gwent NP22 3EL (GB)
(72) Inventor: Morse, Jon, Caerphilly, CF83 2BG (GB); Ahanchian, Nafiseh, Rhondda Cynon Taff, CF72 8PA (GB); Fealey, James, Gwent, NP22 3EL (GB); Cushing, Richard, Caerphilly, NP11 4EN (GB)
(74) Representative: Wynne-Jones IP Limited

(57) **Abstract**

An improved pressure relieving valve assembly is disclosed, together with an inflatable support assembly incorporating the pressure relieving valve assembly. The pressure relieving assembly comprises a valve body having a valve inlet and a valve outlet, and a valve chamber fluidly coupled with the valve inlet and the valve outlet. A plunger is disposed within the chamber and arranged to move within the valve chamber to selectively open and close a passageway through the chamber between the valve inlet and the valve outlet. Embodiments are disclosed in which the assembly further comprises audible and/or visual indicators for indicating whether the passageway is open or closed, for assisting a user when inflating an inflatable support member to an optimum pressure.

## Description

The present invention relates to a pressure relieving valve assembly and an inflatable support assembly. The present invention further relates to a method of inflating an inflatable support member.

### Introduction

Inflatable device supports are currently provided to patients to protect, support and treat parts of a patient's body susceptible to the development of pressure sores and ulcers. The inflatable devices can include cushions and mattresses, and are arranged to minimise the development of pressure sores and ulcers by dispersing the weight of the body part across the surface area of the support. However, the ability of an inflatable device to properly support a patient is critically dependent on the device being inflated to the correct pressure.

WO98/42238 discloses an inflation assembly, namely a pump, for enabling a user to inflate an inflatable device, namely a mattress. The pump includes a pressure relief valve for preventing over-inflating the device to ensure that the device is inflated to the same optimum pressure to achieve a suitable support performance. However, the pump disclosed in WO98/42238 requires careful control by the user to ensure the optimum pressure is achieved. Specifically, the pump must be operated to inflate the device beyond the optimum pressure and when this optimum pressure has been exceeded, excess air is preferentially exhausted to the atmosphere via the valve. This exhaustion of excess air is made known to the user simply by the user hearing the exhaustion, and so this requires a careful monitoring by the user. When the user hears the exhaustion of air, the user is required to stop the inflation and then wait for the pressure relief valve to shut off to effectively prevent further exhaustion. At this point, namely when the user can no longer hear the exhaustion of air and thereby recognising that the exhaustion of air has stopped, the user is required to timely remove the pump to prevent any more air from passing out from the device in the reverse direction through the pump, and thus becoming under-inflated. It is evident therefore that the user is required to carefully monitor the inflation and partial deflation of the device to ensure that the device is suitably inflated. The inflation is thus subject to user awareness of the exhaustion of air from the device and the ability of the user to timely disconnect the pump, and as such it is found that the pressure to which the device is inflated can vary significantly and fall outside an acceptable pressure range. The problem is exacerbated when the inflation is performed in a noisy environment and/or with persons with reduced hearing function as it can be difficult to determine the point at which the optimum pressure has been achieved during the inflation step, and also the point at which the exhaustion of air is shut off during the partial deflation step.

It is further noted that the correct inflation pressure applied to inflatable devices is critically dependent on the correct operation of the pressure relief valve. The pressure relief valve disclosed in WO98/42238 comprises a membrane 12 (see figures 4 and 5 of WO98/42238) which is designed to close off outlet holes 15 within the inflation assembly when the pressure within an inflatable device reaches a threshold pressure. Once the threshold pressure has been reached to enable the membrane to close the outlet holes 15, the back pressure within the inflation assembly causes a valve body 18 to move off a valve seal 19, via a compression of a spring 20, which enables excess air to vent through an outlet passageway 21. The correct operation of the valve is thus dependent on the membrane 12 suitably closing the apertures 15 and also a suitable compression of the spring 20.

When manufacturing the pressure relief valve disclosed in WO98/72238 it is found that the spring 20 can become misaligned with the valve body 18 and become snagged on the spring seat (unlabelled in WO98/42238). This can prevent the valve body from opening the exhaust passage 21 at the required pressure leading to an over-inflation of the device. Moreover, the membrane 12 is constrained to move in a vertical direction to effectively open and close the outlet holes 15, by moving within a cylindrical channel formed by walls of the end cap 9. It is found that the membrane can tilt and snag within the channel leading to an incomplete or incorrect closure of outlet holes 15, which again manifests as an incorrect pressure within the inflatable device.

We have now devised an improved pressure relieving valve assembly, inflation assembly and method of inflating an inflatable support member.

### Statements of the Invention

In accordance with a first aspect of the invention, there is provided a pressure relieving valve assembly for adjusting the pressure to which an inflatable support member is inflated, the assembly comprising:
- a valve body defining a valve chamber;
- the valve body comprising a valve inlet and a valve outlet, the valve inlet and valve outlet being arranged in fluid communication with the valve chamber;
- a plunger arranged to move within the valve chamber to selectively open and close a passageway through the chamber between the valve inlet and the valve outlet;
- the assembly further comprising an indicator for indicating whether the passageway is open or closed.

In an embodiment, the indicator comprises an audible indicator and/or a visual indicator to provide an operator with feedback as to the position of the plunger and thus whether the passageway is open or closed to the passage of air.

In an embodiment, the assembly further comprises a valve housing within which the valve body is disposed, the housing comprising an exhaust duct fluidly coupled with the valve outlet, the exhaust duct extending between the valve outlet and an exhaust outlet.

In an embodiment, the pressure relieving valve assembly further comprises an adapter for coupling with an inflation inlet of an inflatable support member. The adapter can be formed integrally with the valve housing.

In an embodiment, the pressure relieving valve assembly further comprises an inflation passageway via which air exhausted from a pump can pass through the adapter into an inflatable support member via the inflation inlet.

The valve body can comprise a first body portion and a second body portion. The plunger is arranged to contact at least the first body portion when moving within the chamber; the plunger and first body portion being arranged to generate an audible tone when the plunger contacts the first body portion when moving to close the passageway. In an alternative embodiment, the plunger is arranged to contact the first and second body portions when moving within the chamber; the plunger, first body portion and second body portion being arranged to generate an audible tone when the plunger contacts the first and second body portion when moving within the passageway to open and close the passageway. It is envisaged that the contact of the plunger with the first and second body portions may create a ping or ringing tone to alert the operator to the position of the plunger.

In an alternative embodiment, the audible indicator comprises an acoustic chamber disposed proximate the valve outlet, the acoustic chamber being arranged to generate an audible tone during the passage of air through the acoustic chamber.

In an embodiment, the visual indicator comprises an elongate shaft disposed within the exhaust duct, the shaft being moveable between a retracted position corresponding to a closed passageway and an extended position corresponding to an open passageway.

In an embodiment, the shaft is housed substantially within the exhaust duct in the retracted position and at least partially extends out from the exhaust outlet in the extended position. The shaft may comprise a colorful decoration or may otherwise comprise a distinctive color so that the shaft is clearly visible to an operator when in the extended position.

In an embodiment, the shaft is biased into the first position by biasing means, such as a coil spring.

In an embodiment, the shaft comprises a plurality of detents for limiting the movement of the shaft within the exhaust duct between the retracted and extended positions.

In an alternative embodiment, the visual indicator comprises an exhaust flap which is hingedly mounted within the exhaust outlet. The exhaust flap is moveable between a first position corresponding to a closed passageway and a second position corresponding to an open passageway. In the first position, the exhaust flap extends across the exhaust outlet and in the second position, the exhaust flap extends out from the exhaust outlet.

The exhaust flap may comprise a colorful decoration or may otherwise comprise a distinctive color so that the exhaust flap is clearly visible to an operator when in the second position.

In an embodiment, the exhaust flap is biased into the first position by biasing means.

In an embodiment, the plunger is biased to close the passageway by a coil spring disposed within the chamber, the coils of the spring comprising a diameter which increase from a first end to a second end of the spring. The first end of the coil spring is disposed adjacent the plunger and the second end of the coil spring is disposed adjacent the second body portion.

In an embodiment, the assembly further comprises a membrane disposed over the valve inlet, and which is arranged to move relative to the valve inlet to selectively open and close the valve inlet in dependance of a direction of air flow relative to the valve inlet.

In an embodiment, the assembly further comprises at least two stabilizing pins to stabilize the movement of the membrane when moving relative to the valve inlet. The stabilizing pins extend through the membrane to maintain an orientation of the membrane relative to the valve inlet.

In an embodiment, the stabilizing pins extend substantially perpendicular to a plane of the membrane, and are angularly separated around the inlet by less than 90⁰.

In an alternative embodiment, the stabilizing pins are angularly separated around the inlet by less than 45⁰.

In accordance with a second aspect of the invention, there is provided a pressure relieving valve assembly comprising:
- a valve body comprising a first body portion and a second body portion, the first and second body portions defining a valve chamber;
- the first body portion comprising a valve inlet and the second body portion comprising a valve outlet;
- a plunger arranged to move within the valve chamber to selectively open and close a passageway through the chamber between the valve inlet and the valve outlet;
- wherein the plunger is biased to close the passageway by a coil spring disposed within the chamber, the coils of the spring comprising a diameter which increase from a first end to a second end of the spring.

In accordance with a third aspect of the invention, there is provided a pressure relieving valve assembly comprising:
- a valve body comprising a first body portion and a second body portion, the first and second body portions defining a valve chamber;
- the first body portion comprising a valve inlet and the second body portion comprising a valve outlet;
- a plunger arranged to move within the valve chamber to selectively open and close a passageway through the chamber between the valve inlet and the valve outlet;
- a membrane disposed over the valve inlet and arranged to move relative to the valve inlet to selectively open and close the valve inlet in dependance of a direction of air flow relative to the valve inlet;
- at least two stabilizing pins to stabilize the movement of the membrane relative to the valve inlet.

In accordance with a fourth aspect of the present invention, there is provided an inflatable support assembly comprising an inflatable support member for supporting the body or limb of a user, the support member comprising an inflation inlet, the assembly comprising further a container for housing the support member in a deflated state, the container comprising a first and second sleeve, and a pressure relieving valve assembly according to the first, second or third aspects, the first sleeve being open at one end and closed at the other end, the second sleeve being open at one end and closed by the pressure relieving valve assembly at the other end, , the first and second sleeves being arrangable in a nested arrangement to define a pump for inflating the inflatable support member.

In accordance with a fifth aspect of the invention, there is provided a method of inflating an inflatable support member, the method comprising:
- coupling a pump with an inflation inlet of an inflatable support member;
- inflating the inflatable support member using the pump;
- providing a first indication when the pressure within the inflatable support member exceeds a predetermined threshold;
- allowing the pressure within the inflatable support member to reduce;
- providing a second indication when the pressure within the inflatable support member has reduced to the predetermined threshold;
- uncoupling the pump from the inflation inlet of the inflatable support member.

In an embodiment, the method comprises the use of the inflatable support assembly of the fourth aspect.

### Brief Description of the Drawings

Figure 1 is a perspective view of an inflatable support assembly according to an embodiment of the present invention, from a first viewpoint, with the first and second sleeves shown in outline showing the inner facing side of the pressure relieving valve assembly according to a first embodiment of the present invention;
Figure 2 is a perspective view of an inflatable support assembly of figure 1 from a second viewpoint, with the first and second sleeves shown in outline, showing the outer facing side of the pressure relieving valve assembly according to the first embodiment of the present invention;
Figure 3 is a sectional view through the pressure relieving valve assembly of figure1, taken along plane A-A, with a schematic illustration of an inflatable support member fluidly coupled therewith (conical spring and visual indicator not shown);
Figure 4 is a magnified view of the pressure relieving valve assembly illustrated in figure 3 with the conical spring and visual indicator shown in situ, with the visual indicator in a retracted position;
Figure 5a is a magnified view of the pressure relieving valve assembly illustrated in figure 3 with the conical spring and visual indicator shown in situ, with the visual indicator in an extended position;
Figure 5b is a perspective view of the pressure relieving valve assembly according to the first embodiment of the present invention, showing the extension of the visual indicator from the exhaust outlet;
Figure 6a is a sectional view through a pressure relievingvalve assembly according to a second embodiment of the present invention;
Figure 6b is a magnified view of the acoustic chamber of the pressure relieving valve assembly illustrated in figure 6a;
Figure 7a is a sectional view through a pressure relievingvalve assembly according to a third embodiment of the present invention;
Figure 7b is a magnified view of the exhaust duct and exhaust flap of the pressure relieving valve assembly illustrated in figure 7a;
Figure 7c is a perspective view of the outer facing side of the pressure relieving valve assembly according to the third embodiment of the present invention, with the exhaust flap in a first position;
Figure 7d is a perspective view of the outer facing side of the pressure relieving valve assembly according to the third embodiment of the present invention, with the exhaust flap in a second position; and,
Figure 8 is a flow chart illustrating the steps associated with a method of inflating an inflatable support member.

### Detailed Description of Embodiments of the Invention

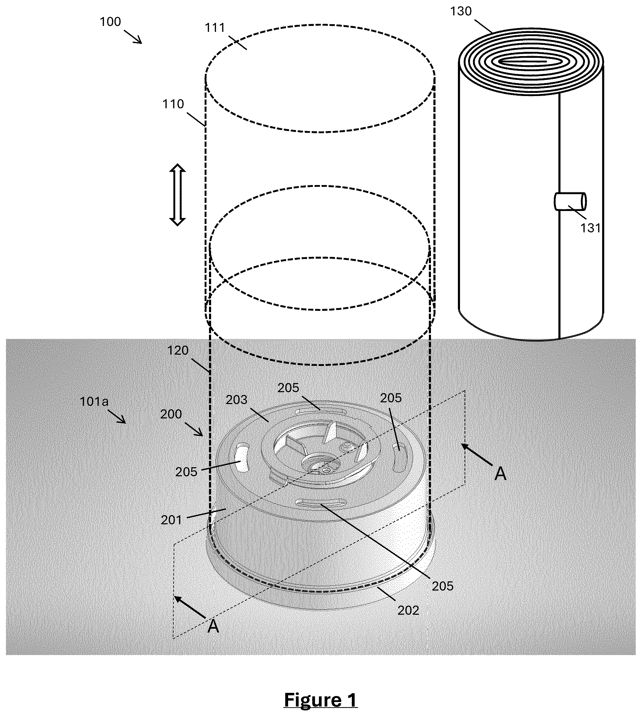

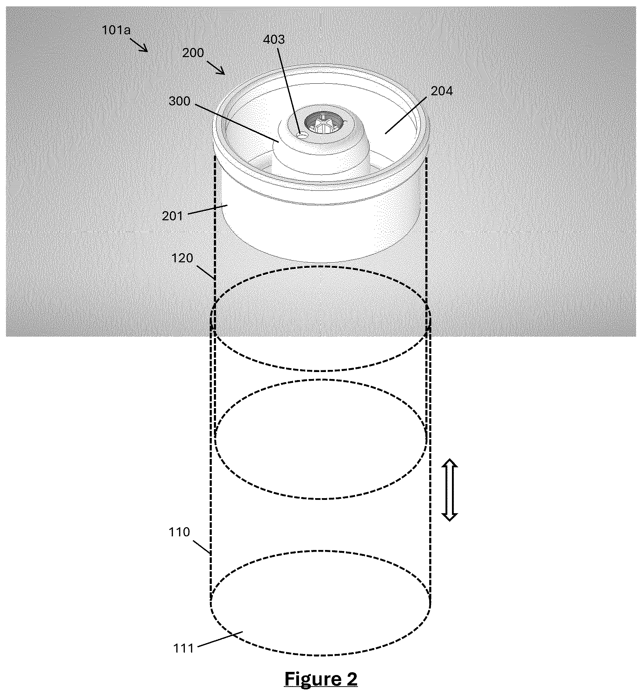

Referring to figures 1 and 2 of the drawings, there is illustrated an inflatable support assembly 100 according to an embodiment of the present invention. The inflatable support assembly 100 comprises a first cylindrical sleeve 110 which may be formed of a plastics material, which is closed at one end by an end wall 111. The opposing end of the first sleeve 110 is open and is arranged to receive a first end of a second cylindrical sleeve 120, which is open at one end (namely, the first end thereof) and closed at the other end by a pressure relieving valve assembly 101a according to a first embodiment of the present invention. The first and second sleeves 110, 120 are arranged to slide relative to each other in a nested or telescopic configuration to create a pump for inflating a support member 130, such as a cushion or mattress. Moreover, the interior of the sleeves 110, 120 are arranged to form a container for housing the inflatable support 130 member in a deflated and rolled configuration. By separating the sleeves 110, 120, the support member 130 can be removed and then the sleeves 110, 120 can be reassembled to create the pump for inflating the support member 130.

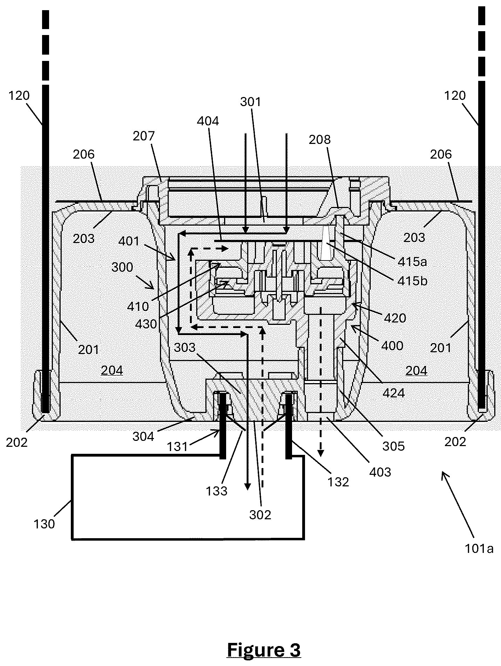

The pressure relieving valve assembly 101a comprises a valve housing 200 which comprises a peripherally extending cylindrical side wall 201, an exterior of which is arranged to extend adjacent the second sleeve 120. A peripheral portion of the side wall 201 is folded radially outwardly of the body, upon itself, to define a peripherally extending channel 202 within which the second end of the second sleeve 120 is arranged to locate. In this respect, it is evident that when the valve assembly 101a is mounted to the second sleeve 120, the valve housing 200 is disposed substantially within the second sleeve 120 and comprises an inner side which faces inwardly of the container defined by the first and second sleeves 110, 120, and an outer side which faces outwardly of the first and second sleeves 110, 120. The pressure relieving valve assembly 101a further comprises a substantially cylindrical shaped valve core 300 concentrically disposed within the valve housing 200 relative to the side wall 201, and spaced relative to the side wall 201 by an annular spacer 203 which extends along an inner side of the valve housing 200, between an inner end of the wall 201 and an inner end of the valve core 300. The side wall 201, annular spacer 203 and valve core 300 may be formed integrally and define a substantially M-shape in cross-section (see figure 3 of the drawings), having an annular cavity 204 formed around the valve core 300, between the core 300 and the side wall 201, which is open to the atmosphere.

The annular spacer 203 comprises a plurality of apertures 205 formed therein (see figure 1), angularly separated around the spacer 203. The apertures 205 provide a direct passage for a fluid, such as an air flow, between the inner and outer sides of the valve assembly 101a. The valve assembly 101a further comprises an inner membrane 206 having an annular shape (see figure 3), which is sized to substantially correspond with the size of the annular spacer 203 and extends upon an inner side of the valve assembly 101a, in use, to cover the apertures 205. The membrane 206 is secured along a radially inward portion thereof to the housing 200 by a fitment 207, such that the membrane 206 can flex along a radial direction to lift off the spacer 203 and open the apertures 205.

The valve core 300 comprises an inlet 301 disposed substantially centrally of the core 300 and the fitment 207, on an inner side of the valve assembly 101a, and an outlet 302 disposed substantially centrally of the core 300 on an outer side of the valve assembly 101a. The outlet 302 is disposed within an adapter 303 formed in an outwardly facing end wall 304 of the valve core 300, for coupling with an inflation inlet 131 of the support member. The inlet 301, outlet 302 and valve core 300 thus define a passageway for a fluid flow such as an airflow, through the valve assembly 101a into the support member 130.

The first and second sleeves 110, 120 are arranged in a nested arrangement and configured for relative movement such that when the first and second sleeves 110, 120 are moved towards each other, the air within the combined volume of the sleeves 110, 120 is compressed. During this compression, the inner membrane 206 is forced upon the spacer 203 to close the apertures 205, so that the compressed air is forced to exit the sleeves 110, 120 into the valve core 300 via the inlet 301, through the passageway and out from the valve core 300 into the support member 130 to inflate the member 130. When the first and second sleeves 110, 120 are moved away from each other, the combined volume of the sleeves 110, 120 increases and reduces the air pressure therein, which causes air to pass from the atmosphere-exposed-cavity 204, through the apertures 205 into the interior of the sleeves 110, 120, by causing the inner membrane 206 to flex inwardly and partially lift off the apertures 205. Accordingly, by repeatedly extending and compressing the sleeves 110, 120, air can be forced into the support member 130 to inflate the member 130.

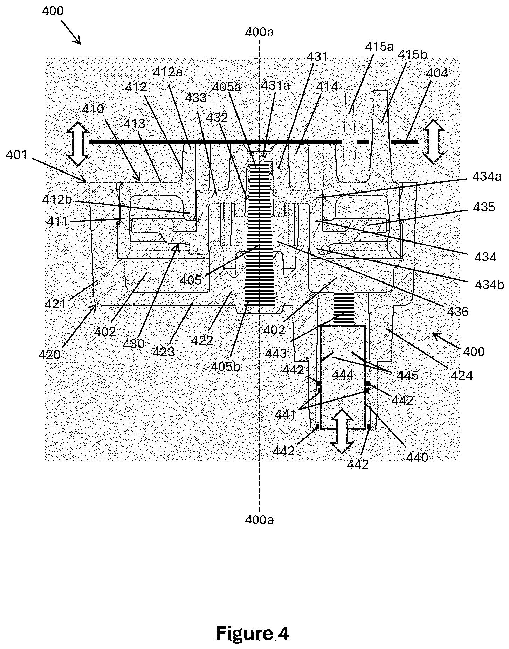

The pressure relieving valve assembly 101a is configured to limit the pressure within the support member 130 to a predefined upper limit using a valve arrangement 400. The valve arrangement 400 comprises a valve body 401 which is mounted within the valve core 300. Specifically, the valve body 401 comprises a first body portion 410 and a second body portion 420, which extend along an axis 400a of the body 401, and which collectively define a valve chamber 402, as illustrated in figure 4 of the drawings.

The second body portion 420 comprises a substantially cylindrical cup section 421 having an open first end, and a collar 422 defining a spring seat formed centrally in an end wall 423 at the second end, which extends inwardly of the cup section 421. The second body portion 420 further comprises an outlet duct 424 formed in the end wall 423, which is radially offset from the collar 422, and which extends toward the end wall 304 of the valve core 300. The cup section 421, collar 422, end wall 423 and outlet duct 424 may be formed integrally, and the outlet duct 424 is arranged to locate within an exhaust duct 305 of the valve core 300. The exhaust duct 305 is similarly radially offset within the valve core 300 relative to the outlet 302 of the core 300, and terminates at an exhaust outlet 403 of the valve assembly 101a.

The first body portion 410 comprises a first cylindrical section 411, having a first diameter, and a second cylindrical section 412 which is open at both ends, having a second diameter which is less than the first diameter. The first and second sections 411, 412 are aligned along a common axis, namely the axis 400a of the body 401 and coupled together by a substantially circular end plate 413 having an aperture (not shown) formed at a central region thereof. The second section 412 extends through the aperture (not shown) and is coupled to the end plate 413 along an inner circumference of the end plate 413 formed by the aperture (not shown), at a position intermediate opposite ends of the second section 412, such that a majority of the second section 412 including an inner end 412a of the second section 412 extends to one side of the plate 413, adjacent the inlet 301 of the valve core 300, while a portion of the second section 412, including an outer end 412b of the second section 412, extends beyond the plate 413 toward the second body portion 420. The first section 411 is coupled at one end thereof to an outer circumference of the plate 413 and extends toward the second body portion 420.

The first section 411, second section 412 and end plate 413 may be formed integrally, and the first diameter is sized to form a friction fit within the open end of the cup section 421 of the second body portion 420. The second section 412 defines an inlet 414 into the valve chamber 402 and the inlet 414 can be selectively opened and closed via a substantially planar outer membrane 404, which extends across the inlet 414.

The valve arrangement 400 further comprises a plunger 430 which is arranged to move within the valve chamber 402 between the first and second body portions 410, 420, along the axis 400a of the body 401. The plunger 430 comprises a collar 431 which is closed by a collar crown 431a at an inwardly facing end thereof, while a second end of the collar 431 extends around an aperture 432 formed in a central region of a first compression plate 433. The diameter of the first compression plate 433 substantially corresponds with an internal diameter of the second section 412 of the first body portion 410.

The plunger 430 further comprises a plunger skirt 434 having a substantially cylindrical shape, an inner end 434a of which is coupled around a circumference of the first compression plate 433, and which extends towards the second body portion 420. The outer diameter of the skirt 434 substantially matches the outer diameter of the first compression plate 433 and as such the outer side of the skirt 434 is arranged to move in sliding relation within the second section 412 of the first body portion 410. An outer end 434b of the skirt 434 is coupled to a second compression plate 435 around a circumference of an aperture 436 formed in the second compression plate 435. The second compression plate 435 comprises an outer diameter which is slightly less than the internal diameter of the first section 411. The collar 431, first compression plate 433, skirt 434 and second compression plate 435 may be formed integrally and are aligned along the axis 400a of the body 401. An internal diameter of the skirt 434 is sized to substantially match an outer diameter of the collar or seat 422 so that the seat 422 can locate within the skirt 434 as the plunger 430 moves relative to the first and second body portions 410, 420.

The plunger 430 is arranged to move between a first and second position within the valve body 401 and the extent to which the plunger 430 can move within the valve body 401, namely the first and second positions, is limited by the length of the skirt 434 and a longitudinal separation of the first and second compression plate 433, 435. In the first position, an inwardly facing side of the second compression plate 435 is arranged to contact the outer end 412b of second section 412, while in the second position, an outer end 434b of the skirt 434 is arranged to contact the end wall 423 of the second body portion 420. The length of the skirt 434, and the extent to which the outer end 412b of the second section 412 extends beyond the end plate 413 is chosen such that when the plunger 430 adopts the second position, the first compression plate 433 extends longitudinally beyond the outer end 412b of the second section 412 to define a passageway from the inlet 301, around the outer end 412b of the second section 412 and around an outer peripheral edge of the second compression plate 435, between the second plate 435 and the cup section 421 of the second body portion 420, towards the exhaust outlet 403, via the outlet duct 424 and exhaust duct 305.

In the first embodiment of the pressure relieving valve assembly 101a, the plunger 430, first body portion 410 and second body portion 420 are formed of a material which is different to the material from which the valve housing 200 may be formed. For example, the valve housing 200 may be formed of a rigid plastics material, whereas the valve body 401 may be formed of a metallic material. The first and second body portions 410, 420 are arranged to form a "bell" type housing such that as the plunger 430 moves between the first and second positions, the contact of the plunger 430 upon the first body portion 410 and/or the second body portion 420 is arranged to generate a ringing tone or similar, to provide an audible indication when the plunger 430 moves to open and close the passageway within the valve body 401. Moreover, the ringing tone generated when the plunger 430 strikes the first body portion 410 may be different to the ringing tone generated when the plunger 430 strikes the second body portion 420 to provide a further indication whether the passageway is open or closed.

The plunger 430 is biased to adopt the first position and thus close the passageway through the valve body 401 by a compression spring 405. In an embodiment, the coils of the spring 405 increase from a first end 405a, namely the end which is arranged to located within the collar 431 of the plunger 430 adjacent the collar crown 432, toward a second end 405b, namely the end which is arranged to locate within the spring seat 422 of the second body portion 420. The spring 405 is orientated to extend along the axis 400a of the valve body 401 and may comprise a conical shape. The increased diameter of the spring coils in the seat 422 compared with the diameter of the spring coils within the collar 431, provide for a more stable spring configuration when assembling the valve body 401 to ensure that the spring 405 is correctly orientated relative to the plunger 430.

The outer membrane 404 extends across the inlet 414 to the valve body 401 and is arranged to form a seal with an inner end 412a of the second section 412 of the first body portion 410. The membrane 404 is held aligned over the inlet 415 to the valve body 401 by at least two stabilizing pins 415a, 415b which extend from the end plate 413 toward the inlet 301 to the valve core 300. At least one of the pins 415b is arranged to locate within a recess 208 formed within the fitment 207 to prevent the membrane 404 from completely moving off the inlet 414 to the valve assembly 400. Moreover, the location of the at least one stabilizing pin 415b within the recess 208 on the fitment 207 serves to further stabilize the valve body 401 within the valve core 300.

The stabilizing pins 415a, 415b can be formed integrally with the end plate 413 and extend substantially perpendicular to the end plate 413. The pins 415a, 415b are further angularly separated around the end plate 413 by less than 90⁰ and more preferably less than 45⁰, and are arranged to pass within locating apertures or slots 406 within the outer membrane 404 so that the alignment and orientation of the membrane 404 can be maintained as the membrane 404 moves relative to the inlet 414 to the valve body 401.

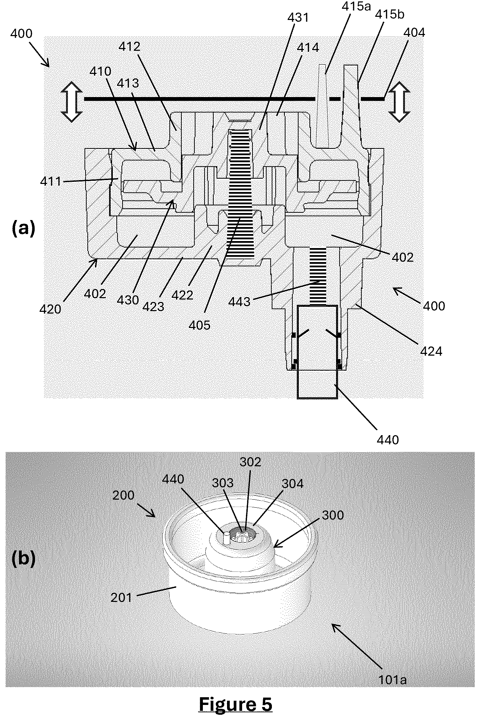

In the first embodiment, the outlet duct 424 and exhaust duct 305 comprises a shaft 440 having an outer diameter which is sized such that the shaft 440 can move relative to the duct 424 and the exhaust duct 305. The shaft 440 comprises a plurality of abutments 441 formed on an exterior thereof which are arranged to contact a plurality of detents 442 formed along an interior of the outlet duct 424, to limit the extent to which the shaft 440 can move within the duct 424 and the exhaust duct 305. The shaft 440 is biased to adopt a retracted position in which the shaft 440 is completely contained and housed within the outlet duct 424 and the exhaust exhaust 305, by a coil spring 443, but is permitted to move partially out from the exhaust outlet 403 of the valve assembly 400, to an extended position, as illustrated in figure 5a and 5b of the drawings. An exterior of the shaft 440 may comprise a colorful decoration so that it is clearly evident when the shaft 440 extends from the outlet 403.

The shaft 440 further comprises a through bore 444 having one or more baffles 445 formed therein which are arranged to catch a portion of an airflow passing out through the outlet 403 of the valve assembly to urge the shaft 440 to move out from the exhaust outlet 403. In a further embodiment, the baffles 445 may be configured to create a waveguide for generating an audible tone, akin to the passage of air through a whistle (not shown) or a wind instrument (not shown), as the air flows along the bore 444 of the shaft 440 to provide an audible indication of the exhaustion of air from the outlet 403.

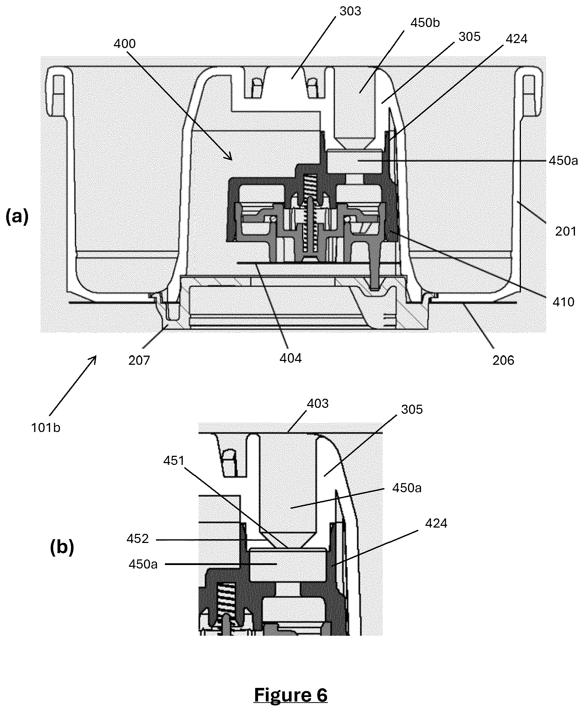

Referring to figure 6 of the drawings, there is illustrated a second embodiment of a pressure relieving valve assembly 100b comprising an alternative audible indicator. Features common to the first and second embodiment have been shown with the same reference numerals. However, in the second embodiment, the first and second body portions 410, 420 of the valve body 401, and the plunger 430, may be formed of a plastics material. Moreover, the shaft 440 with the associated abutments 441, detents 442 and coil spring 443 have been removed. In the second embodiment, the outlet duct 424 and exhaust duct 305 collectively define an acoustic chamber 450 which comprises a first portion 450a that is defined by the outlet duct 424 and a second portion 450b which is defined by the exhaust duct 305. The cross-sectional area of the first portion 450a is substantially uniform along the length thereof, however, at the interface between the first and second portions 450a, 450b, there is a discontinuous change in cross-sectional area. The interface is defined by an entrance port 451 which comprises a narrow central aperture (compared with the cross-sectional area of the first portion 450a), the diameter of which increases in a direction which is into the second chamber 450b. In this respect, the transition from the first chamber portion to the second chamber portion comprises a tapered duct profile 452 to create a resonant style cavity for generating an audible tone, as air passes from the first portion 450a to the second portion 450b of the acoustic chamber 450.

Accordingly, when the passageway through the valve body 401 is open, air is permitted to pass along the acoustic chamber 450 and out through the exhaust outlet 403. This passage of air causes the acoustic chamber to generate an audible "whistle" tone which can be detected by a user when inflating an inflatable support member 130 and used to indicate when to stop inflating. Once the passageway subsequently closes, the airflow along the acoustic chamber 450 will be stopped and the audible whistle will cease, thereby providing a further indication to the user when to disconnect the valve assembly 101b from the inflation inlet 131.

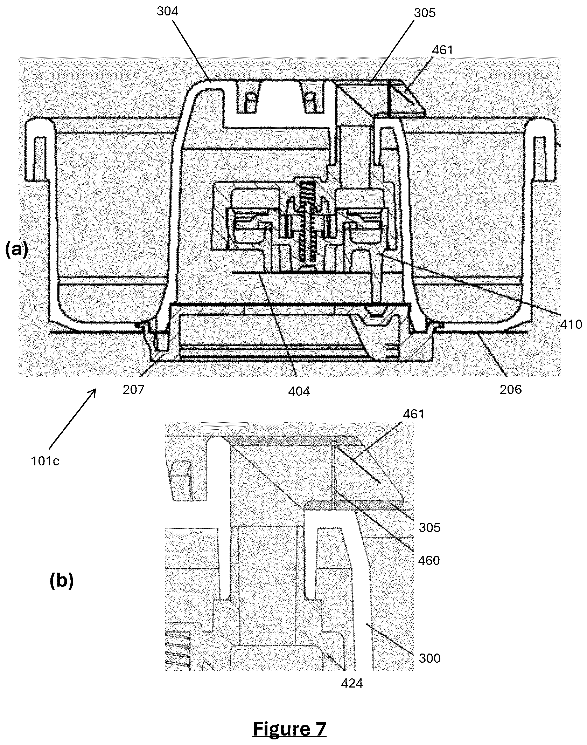

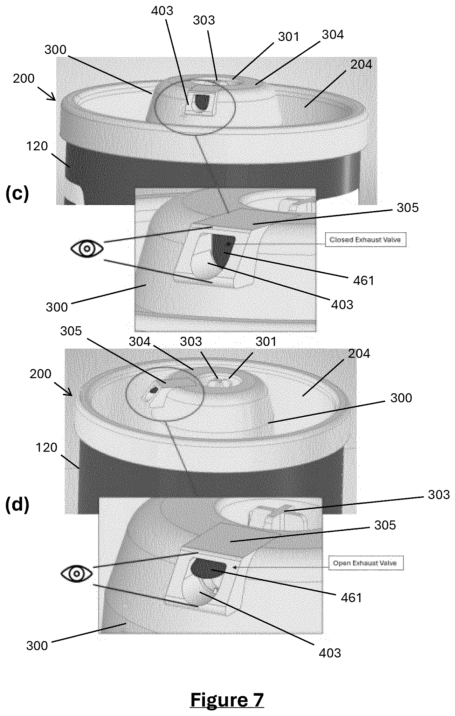

Referring to figure 7 of the drawings, there is illustrated a third embodiment of a pressure relieving valve assembly 101c comprising an alternative visual indicator. Features common to the first and third embodiment have been shown with the same reference numerals. However, in the third embodiment, the first and second body portions 410, 420 of the valve body 401, and the plunger 430, may be formed of a plastics material. Moreover, the shaft 440 with the associated abutments 441, detents 442 and coil spring 443 have been removed. Moreover, the exhaust duct 305 is turned through substantially 90⁰ on the end wall 304 of the valve core 300, such that the exhaust outlet 403 extends substantially perpendicular to the end wall 304 (rather than in the plane of the end wall, as with the first embodiment).

The valve assembly 101c of the third embodiment further comprises an exhaust frame 460 which extends across and within the exhaust duct 305 proximate the exhaust outlet 403. The frame 460 is arranged to support an exhaust flap 461 which is hingedly mounted to the frame 460. The flap 461 comprises a planar shape, a periphery of which substantially conforms to the internal shape of the exhaust duct 305. The flap 461 is biased to a first position (under the influence of a spring for example, not shown) in which the flap 461 extends within the frame 460 and substantially within the exhaust duct 403, but can pivot about the hinge, under the influence of an air flow along the exhaust duct, to pivot to a second position in which a substantial portion of the flap 461 extends out from the exhaust outlet 403. The flap 461 comprises a colorful or otherwise distinctive decoration so that the exhaust flap 461 is clearly visible to an operator when in the second position.

Accordingly, when the passageway through the valve body 401 is open, air is permitted to pass along the exhaust duct 305 and out through the exhaust outlet 403. This passage of air causes the flap 461 to pivot to the second position so that it becomes visible to an operator of the pump for example when inflating an inflatable support member 130, which can be used to indicate when to stop inflating. Once the passageway closes, the airflow along the exhaust duct 305 stops and the flap 461 returns to the first position within the exhaust duct 305 (owing to the bias on the spring (not shown)), and thus substantially from view by the user thereby providing a further indication to the user when to disconnect the valve assembly 101b from the inflation inlet 131.

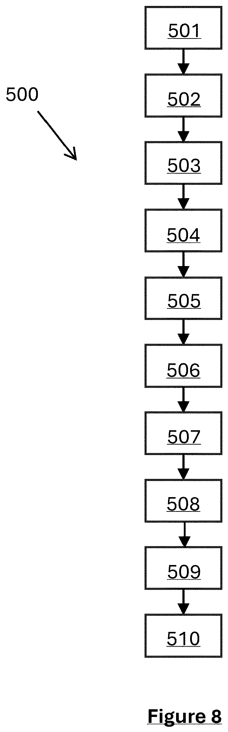

Referring to figure 8 of the drawings there is illustrated the steps associated with a method 500 of inflating an inflatable support member according to an embodiment of the present invention. In use, an operator (not shown) first separates the first and second sleeves 110, 120 of the inflatable support assembly 100 comprising a pressure relieving valve assembly 101a of the first aspect at step 501 by pulling the sleeves 110, 120 apart, and removes the rolled and deflated support member 130 at step 502. The operator then reassembles the sleeves 110, 120 to the nested configuration and unrolls the support member 130 at step 503. The operator subsequently couples an inflation inlet 131 of the support member 130 with the adapter 303 of the valve core 300 on the valve assembly 101a at step 504. The inflation inlet 131 comprises a cylindrical housing 132 with a one-way valve 133 located therein. A diameter of the cylindrical housing 132 is sized to match the adapter 303 such that when the adapter 303 is inserted into the housing 132 of the inlet 131, the one-way valve is held opened to allow air to pass between the adapter 303 and the interior of the support member 130.

Once the inflation inlet has been suitably coupled with the adapter at step 504, the operator can extend the nested sleeve arrangement to draw air into the sleeves 110, 120via the apertures 205 formed on the spacer 203 of the valve housing 200. The operator then pushes the sleeves 110, 120 together to force the air contained therein to pass into the valve core 300 via the inlet 301. This movement of air into the inlet 301 impinges on an inner side of the outer membrane 404 which forces the outer membrane 400 onto the inner end 412a of the second section 412 to effectively seal and close the inlet 414 to the valve body 401. Accordingly, air passing from the sleeves 110, 120 into the valve core 300 through the inlet 301 is arranged to bypass the valve body 401 and pass out from the outlet 302 in the adapter 303 to the support member 130 to inflate the member 130 (as illustrated with the solid arrow lines in figure 3). The relative movement of the sleeves 110, 120 is repeated at step 505 to progressively increase the air pressure within the support member 130.

However, when the air pressure within the support member 130 reaches a threshold pressure, the back pressure created within the valve core 300 (as illustrated with the dashed arrow lines in figure 3) will cause the outer membrane 404 to lift off the inlet 414 to the valve body 401 and open the inlet 414. The opening of the inlet 414 thus exposes the plunger 430 to the back pressure which results in the plunger 430 moving towards the second body portion 420 from the first position to the second position, and in doing so, compressing the coil spring 405. When the plunger 430 reaches the second position, the plunger 430 contacts the second body portion 420 which generates an audible ringing tone at step 506, to inform the operator that the pressure relieving valve assembly 400 has been opened and that the support member 130 has been inflated beyond the optimum pressure. In addition, the opening of the valve assembly 400 causes the excess air to pass out through the valve body 401 and the outlet 403, via the outlet duct 424 and exhaust duct 305. The air flow passing along the outlet duct 424 and exhaust duct 305 interacts with the baffles 445 within the shaft 440 and generates a force on the shaft 440 to cause the shaft 440 to extend out from the outlet 403 against the bias of the spring 443 at step 507. Accordingly, the opening of the passageway within the valve body 401 is made known to the operator by the audible ringing tone and also by the visual indication of the shaft 440 extending from the end wall 304 of the end cap 200. In addition, it is envisaged that the air passing through the shaft 440 may generate an audible tone similar to a whistle or wind instrument to further alert the operator that the passageway of the valve body 401 has been opened and to stop further inflation of the support member at step 508.

When the operator stops inflating at step 508, the air pressure within the support member 130will begin to reduce owing to the inflation inlet 131 being held open by virtue of its coupling with the adapter 303. However, once the pressure in the support member 130 reduces to the optimum pressure, the back pressure created within the valve assembly 400 will reduce. At a threshold condition (namely when the pressure within the support member reaches the optimum pressure), the bias of the coil spring 405 will overcome the back pressure on the plunger, causing the plunger 430 to move from the second position back to the first position, thereby closing the passageway through the valve body 401. This movement to the first position is made known to the operator by a further ringing tone as the plunger 430 strikes the first body portion 410 at step 509, and further by the retraction of the shaft back into the end cap at step 510, owing to the bias of spring 443. When the operator recognizes that the valve assembly has shut-off, the operator can then disconnect the inflation inlet 131 of the support member 130 from the adapter 303 at step 510. This results in the valve 133 within the housing 132 of the inflation inlet 131 closing to prevent any further outflow of air from the support member 130 so that the pressure within the support member 130 is maintained at the optimum pressure.

The skilled reader will recognize that this method 500 can also be employed with an inflation support assembly 100 comprising a pressure relieving valve assembly of the second and third embodiments. The opening and closing of the passageway within the valve body 401 is made known to the operator by the audible "whistle" tone of the acoustic chamber 450 of the assembly 101b of the second embodiment, and also by the visible movement of the exhaust flap 461 in the of the assembly 101c of the third embodiment. These audible and visual indicators similarly enable an operator to inflate an inflatable support member to the optimum pressure.

From the foregoing it is evident that the pressure relieving valve assembly 101a, 101b, 101c, inflation assembly 100 and method 500 of inflating an inflatable support member 130 provides for an improved inflation of support members and thus an improved performance of the support members in use.

## Claims

1. A pressure relieving valve assembly for adjusting the pressure to which an inflatable support member is inflated, the assembly comprising:
- a valve body defining a valve chamber;
- the valve body comprising a valve inlet and a valve outlet, the valve inlet and valve outlet being arranged in fluid communication with the valve chamber;
- a plunger arranged to move within the valve chamber to selectively open and close a passageway through the chamber between the valve inlet and the valve outlet;
- the assembly further comprising an indicator for indicating whether the passageway is open or closed.

2. A pressure relieving valve assembly according to claim 1, further comprising a valve housing within which the valve body is disposed, the housing comprising an exhaust duct fluidly coupled with the valve outlet, the exhaust duct extending between the valve outlet and an exhaust outlet.

3. A pressure relieving valve assembly according to any preceding claim, wherein the valve body comprises a first body portion and a second body portion.

4. A pressure relieving valve assembly according to any preceding claim, wherein the indicator comprises an audible indicator.

5. A pressure relieving valve assembly according to any preceding claim, wherein the indicator comprises or further comprises a visual indicator.

6. A pressure relieving valve assembly according to claim 4 when dependent on claim 2, wherein the plunger is arranged to contact at least the first body portion when moving within the chamber, the plunger and first body portion being arranged to generate an audible tone when the plunger contacts the first body portion when moving to close the passageway.

7. A pressure relieving valve assembly according to claim 4, wherein the audible indicator comprises an acoustic chamber disposed proximate the valve outlet, the acoustic chamber being arranged to generate an audible tone during the passage of air through the acoustic chamber.

8. A pressure relieving valve assembly according to claim 5 when dependent on claim 3, wherein the visual indicator comprises an elongate shaft disposed within the second body portion, the shaft being moveable between a retracted position corresponding to a closed passageway and an extended position corresponding to an open passageway.

9. A pressure relieving valve assembly according to claim 5, when dependent on claim 2, wherein the visual indicator comprises an exhaust flap which is hingedly mounted within the exhaust outlet.

10. A pressure relieving valve assembly according to claim 9, wherein the exhaust flap is moveable between a first position corresponding to a closed passageway within the valve body and a second position corresponding to an open passageway within the valve body.

11. A pressure relieving valve assembly according to claim 10, wherein in the first position, the exhaust flap extends across the exhaust outlet and in the second position, the exhaust flap extends out from the exhaust outlet.

12. A pressure relieving valve assembly according to claim 10 or 11, wherein the exhaust flap is biased into the first position by biasing means.

13. A pressure relieving valve assembly according to any preceding claim, further comprising a membrane disposed over the valve inlet and arranged to move relative to the valve inlet to selectively open and close the valve inlet in dependance of a direction of air flow relative to the valve inlet.

14. An inflatable support assembly comprising an inflatable support member for supporting the body or limb of a user, the support member comprising an inflation inlet, the assembly further comprising a container for housing the support member in a deflated state, the container comprising a first and second sleeve and a pressure relieving valve assembly according to any preceding claim, the first sleeve being open at one end and closed at the other end, the second sleeve being open at one end and closed by the pressure relieving valve assembly at the other end, the first and second sleeves being arrangable in a nested arrangement to define a pump for inflating the inflatable support member.

15. A method of inflating an inflatable support member, the method comprising:
- coupling a pump with an inflation inlet of an inflatable support member;
- inflating the inflatable support member using the pump;
- providing a first indication when the pressure within the inflatable support member exceeds a predetermined threshold;
- allowing the pressure within the inflatable support member to reduce;
- providing a second indication when the pressure within the inflatable support member has reduced to the predetermined threshold;
- uncoupling the pump from the inflation inlet of the inflatable support member.
